# EUROPEAN PATENT APPLICATION

(11) **EP 1 440 682 A1**
(43) Date of publication of application: **28.07.2004**
(21) Application number: 02783101.5
(22) Date of filing: 22.10.2002
(51) Int. Cl.: A61K 7/36, A61K 9/107, A61K 31/53, A61K 33/30

(54) **COMPOSITION IN THE FORM OF AN EMULSION THAT CAN BE USED TO TREAT PLANTAR BROMHIDROSIS AND/OR HYPERHIDROSIS**

(30) Priority: 31.10.2001 ES 200102409
(71) Applicant: Quimversion, S.L., 50008 Zaragoza (ES)
(72) Inventor: TANCO SALAS, Jesús, E-50008 Zaragoza (ES)
(74) Representative: PROPI, S.L.
(86) International application number: PCT/ES2002/000498
(87) International publication number: WO 2003/037286

(57) **Abstract**

Said improvements consist of improved emulsion form compositions comprising hexamethylenetetramine or a derivative thereof and zinc oxide, which can be used as body deodorant.

## Description

### FIELD OF THE INVENTION

This invention is generally related to improvements introduced in the object of Spanish patent application number P9800863. This invention particularly refers to emulsion form deodorant compositions comprising an aqueous phase containing hexamethylenetetramine or a derivative thereof, and an oily phase containing zinc oxide.

### BACKGROUND OF THE INVENTION

The topical application of deodorant products for the purpose of suppressing unpleasant odors is a well known fact. In this sense, numerous deodorant products in solid or liquid composition forms are known. In spite of this, there is still the need for developing new deodorant products for the purpose of increasing the user's choice capacity.

Spanish patent application number P9800863 discloses an emulsion form composition, useful for the elaboration of cosmetic or pharmaceutical products intended for the treatment of plantar hyperhidrosis and/or bromhidrosis, comprising an oily phase containing between 2% and 6% by weight of zinc oxide with regard to the total composition weight, and an aqueous phase comprising between 2% and 6% by weight of hexamethylenetetramine or a derivative thereof with regard to the total composition weight. Now it has been found that such compositions as well as the compositions resulting from varying the concentrations of said components in their respective phases, within determined intervals, can be used in the elaboration of body deodorants.

### DETAILED DESCRIPTION OF THE INVENTION

In one aspect, the present invention refers to improvements introduced in the object of Spanish patent application number P9800863 regarding an emulsion form composition, useful, among other things, in the elaboration of cosmetic products, comprising an oily phase containing zinc oxide and an aqueous phase containing hexamethylenetetramine or a derivative thereof, said processes being characterized in that the aqueous phase comprises between 0.05% and 10% by weight of hexamethylenetetramine or a derivative thereof with regard to the total composition weight, and the oily phase comprises between 0.1% and 10% by weight of zinc oxide with regard to the total composition weight, with the proviso that when the concentration of hexamethylenetetramine or derivative thereof in the aqueous phase is comprised between 2% and 6% by weight with regard to the total composition weight, then the zinc oxide concentration in the oily phase is not comprised between 2% and 6% by weight with regard to the total composition weight.

Therefore, the present invention provides an emulsion form composition suitable for the elaboration of a deodorant product, hereinafter composition of the invention, comprising
a) an aqueous phase comprising hexamethylenetetramine or a derivative thereof, in an amount comprised between 0.05% and 10% by weight with regard to the total weight, an
b) an oily phase comprising zinc oxide in an amount comprised between 0.1% and 10% by weight with regard to the total composition weight,
with the proviso that when the concentration of hexamethylenetetramine or derivative thereof in the aqueous phase is comprised between 2% and 6% by weight with regard to the total composition weight, then the zinc oxide concentration in the oily phase is not comprised between 2% and 6% by weight with regard to the total composition weight.

In a particular embodiment, the concentration of hexamethylenetetramine or derivative thereof in the aqueous phase is lower than 2%, preferably lower than 1% by weight, with regard to the total composition weight. In a specific embodiment, the concentration of hexamethylenetetramine or derivative thereof in the aqueous phase is 0.7% by weight with regard to the total composition weight.

In another particular embodiment, the zinc oxide concentration in the oily phase is lower than 2%, preferably lower than 1% by weight, with regard to the total composition weight. In a specific embodiment, the zinc oxide concentration in the oily phase is 0.5% by weight with regard to the total composition weight.

The aqueous phase of the composition of the invention contains an aqueous solution of hexamethylenetetramine or derivative thereof, together with, optionally, a water-miscible organic solvent, such as a polyol, for example propylene glycol, or other components, for example, sodium chloride, stabilizers, etc.

The oily phase of the composition of the invention contains at least an oil or oil mixture, in which the zinc oxide is dispersed. The oils which can be used in the oily phase of the composition of the invention can be animal oils, vegetable oils, mineral oils, synthesis oils, silicon oils (volatile or non-volatile) and, generally, any oil suitable for the elaboration of emulsion form cosmetic compositions.

In a particular embodiment, the oily phase comprises either an emulsifiable fat or a non-emulsifiable fat and an emulsifier. In this case, the composition of the invention can be found in an oily-aqueous (O/A) or aqueous-oily (A/O) emulsion form, depending, among other factors, on the hydrophilic-lipophilic balance (HLB) of the emulsifier or emulsifiable fat used. Generally, emulsifiers or emulsifiable fats with HLB values comprised between 4 and 7 yield A/O type emulsions, whereas emulsifiers or emulsifiable fats with HLB values comprised between 8 and 18 yield O/A type emulsions.

In another particular embodiment, the oily phase comprises one or more volatile or non-volatile silicone oils, leading to the so-called silicone emulsions, which can furthermore contain, optionally, a silicone emulsifier. Illustrative examples of silicones which can be used in the oily phase of the silicone emulsions provided by this invention include dimethylsiloxane-type cyclical or linear silicones, for example dimethicone, cyclomethicone, etc. The silicone emulsifier can be a polydiorganosiloxane, such as, for example, a dimethicone copolyol and cyclomethicone mixture.

The composition of the invention can be easily obtained by conventional methods, which generally comprise preparing the aqueous and oily phases separately and mixing said phases by stirring until formation of the emulsion.

In a particular embodiment, the emulsions in which the oily phase comprises either an emulsifiable fat or a non-emulsifiable fat and an emulsifier can be advantageously obtained by means of a process comprising the steps of:
i) preparing the oily phase by mixing the oily phase components and heating to a temperature equal to or slightly higher than the melting point of the component with the highest melting point, typically between 5°C and 10°C above the melting point of the component with the highest melting point;
ii) separately preparing the aqueous phase by mixing the aqueous phase components and heating to the same temperature as the oily phase, and,
iii) mixing the oily phase with the aqueous phase and stirring until the emulsion is formed.

Silicone emulsions can be easily prepared by means of a process comprising the steps of:
i) on one hand, preparing the silicone phase by means of the mixture of its components,
ii) separately preparing the aqueous phase by means of the mixture of its components, and,
iii) mixing said silicone and aqueous phases and stirring until the silicone emulsion is formed.

In this case, it is not necessary to heat any of the phases, therefore said silicone phases can be prepared at room temperature, for example, between 15°C and 30°C.

The composition of the invention is useful for the elaboration of cosmetic products, particularly deodorant products, for example body deodorants and/or foot deodorants. In addition to the composition of the invention, said cosmetic products can contain one or several additives, in any of the phases, of those normally used in the elaboration of deodorant products, providing certain features, for example, aromas, antioxidants, stabilizers, hydrates, antiseptics, etc.

The deodorant and antiperspirant capacity of the composition of the invention is based on the inhibiting effect on the bacterial flora of the skin which hexamethylenetetramine has and on the astringent and absorbent action of zinc oxide.

Zinc oxide is an excellent product for the treatment of hyperhidrosis and bromhidrosis, whereas hexamethylenetetramine is an excellent product against bromhidrosis and as an antiseptic suitable for eliminating sweat putrefaction microorganisms. In order to obtain a synergic action of both products, it is necessary to join them in a same preparation, but then the problem of chemical interaction between them occurs since hexamethylenetetramine is very reactive with metal ions, for example with the zinc ions released by the zinc oxide, with which it forms complexes or chelates. Due to this interaction between both components, on one hand a reduction of the zinc action occurs (since some zinc ions are complexed and sequestered by hexamethylenetetramine) and, on the other hand, a reduction in the chemical stability of hexamethylenetetramine occurs, the latter thereby decomposes into formaldehyde and urea (irritating and toxic), which increases the irritating and sensitizing action. Furthermore, this reduction in chemical stability of hexamethylenetetramine causes a decrease of its concentration at the time of its application and, accordingly, a reduction in terms of its effectiveness. By separating both ingredients into different emulsion phases, its synergic action is achieved without the losses caused by their chemical interaction and, therefore, by delaying and decreasing the decomposition of hexamethylenetetramine into formaldehyde and urea, less skin irritation and sensitization and a longer-lasting deodorant effect are achieved. The composition of the invention therefore provides a greater deodorant effect at equal active ingredient doses than other preparations containing both ingredients in the same phase, or an equal deodorant effect but with a smaller dose. Therefore, an advantage of the composition of the invention is that it lacks unwanted cutaneous irritation and sensitization side effects (pruritus, itching, tetters, etc.) associated with other compositions containing said active ingredients.

The following example serves to illustrate a specific way to carry out the invention without it being necessarily considered limiting of the scope thereof.

### EXAMPLE

### Elaboration of a deodorant silicone emulsion

A deodorant silicone emulsion is prepared according to the processes disclosed below.

### Process I

A mixture formed by 9.5 parts by weight of dimethicone copolyol and cyclomethicone is added over a mixture formed by 15 parts by weight of cyclomethicone, 7.5 parts by weight of vaseline, 0.5 parts by weight of zinc oxide and 0.5 parts by weight of triclosan and stirred vigorously.

Then, a mixture formed by 0.5 parts by weight of prenonip and 9 parts by weight of propylene glycol is added to the resulting mixture and stirred vigorously. Subsequently, a mixture formed by 1.6 parts by weight of sodium chloride and water (sufficient quantity for 100 parts by weight) is added over the resulting mixture and stirred vigorously. Finally, a mixture formed by 0.7 parts by weight of hexamethylenetetramine, 1.6 parts by weight of sodium chloride and water (sufficient quantity for 100 parts by weight) is added over the resulting mixture and stirred vigorously until the emulsion is formed.

### Process II

A mixture formed by 0.5 parts by weight of prenonip and 9 parts by weight of propylene glycol is added over a mixture formed by 9.5 parts by weight of dimethicone copolyol and cyclomethicone and stirred vigorously. Then, a mixture formed by 1.6 parts by weight of sodium chloride and water (sufficient quantity for 100 parts by weight) is added over the resulting mixture and stirred vigorously. Then, a mixture formed by 0.7 parts by weight of hexamethylenetetramine, 1.6 parts by weight of sodium chloride and water (sufficient quantity for 100 parts by weight) is added over the resulting mixture and stirred vigorously until the emulsion is formed. Finally, the resulting mixture is added over a mixture formed by 15 parts by weight of cyclomethicone, 7.5 parts by weight of vaseline, 0.5 parts by weight of zinc oxide and 0.5 parts by weight of triclosan and stirred vigorously until the emulsion is formed.

## Claims

1. Improvements introduced in the object of Spanish patent application number P9800863 related to an emulsion form composition, useful, among other things, for the elaboration of cosmetic products, comprising an oily phase containing zinc oxide and an aqueous phase containing hexamethylenetetramine or derivative thereof, said processes being **characterized in that** the aqueous phase comprises between 0.05% and 10% by weight of hexamethylenetetramine or a derivative thereof with regard to the total composition weight, and the oily phase comprises between 0.1% and 10% by weight of zinc oxide with regard to the total composition weight, with the proviso that when the concentration of hexamethylenetetramine or derivative thereof in the aqueous phase is comprised between 2% and 6% by weight with regard to the total composition weight, then the zinc oxide concentration in the oily phase is not comprised between 2% and 6% by weight with regard to the total composition weight.

2. An emulsion form composition comprising:
a) an aqueous phase comprising hexamethylenetetramine or a derivative thereof, in an amount comprised between 0.05% and 10% by weight with regard to the total weight, and
b) an oily phase comprising zinc oxide in an amount comprised between 0.1% and 10% by weight with regard to the total composition weight,
with the proviso that when the concentration of hexamethylenetetramine or derivative thereof in the aqueous phase is comprised between 2% and 6% by weight with regard to the total composition weight, then the zinc oxide concentration in the oily phase is not comprised between 2% and 6% by weight with regard to the total composition weight.

3. A composition according to claim 2, in which the concentration of hexamethylenetetramine or derivative thereof in the aqueous phase is lower than 2%, preferably, lower than 1% by weight with regard to the total composition weight.

4. A composition according to claim 3, in which the concentration of hexamethylenetetramine or derivative thereof in the aqueous phase is 0.7% by weight with regard to the total composition weight.

5. A composition according to claim 2, in which the zinc oxide concentration in the oily phase is lower than 2%, preferably, lower than 1% by weight with regard to the total composition weight.

6. A composition according to claim 5, in which the zinc oxide concentration in the oily phase is 0.5% by weight with regard to the total composition weight.

7. A composition according to claim 2, in which the aqueous phase contains an aqueous solution of hexamethylenetetramine or derivative thereof, together with, optionally, a water-miscible organic solvent.

8. A composition according to claim 2, in which the oily phase contains at least an oil or oil mixture in which the zinc oxide is dispersed.

9. A composition according to claim 8, in which said oils can be animal oils, vegetable oils, mineral oils, synthesis oils or silicone oils.

10. A composition according to claim 2, in which the oily phase comprises an emulsifiable fat or a non-emulsifiable fat and an emulsifier.

11. A composition according to claim 2, in which the oily phase comprises one or more volatile or non-volatile silicone oils, together with, optionally, a silicone emulsifier.

12. A composition according to claim 11, in which said silicone oil comprises one or more dimethylsiloxane-type cyclical or linear silicones.

13. A composition according to claim 12, in which said silicone oil comprises dimethicone or cyclomethicone.

14. A composition according to claim 11, in which said silicone emulsifier is a polydiorganosiloxane.

15. A composition according to claim 14, in which said silicone emulsifier is dimethicone copolyol and cyclomethicone mixture.

16. A process for obtaining an emulsion form composition according to claim 2, comprising separately preparing the aqueous and oily phases and mixing said phases by stirring until formation of the emulsion.

17. A process according to claim 16 for obtaining an emulsion in which the oily phase comprises an emulsifiable fat or non-emulsifiable fat and an emulsifier, comprising the steps of:
i) preparing the oily phase by mixing the oily phase components and heating to a temperature equal to or higher than the melting point of the component with the highest melting point;
ii) separately preparing the aqueous phase by mixing the aqueous phase components and heating to the same temperature as the oily phase, and,
iii)mixing the oily phase with the aqueous phase and stirring until the emulsion is formed.

18. A process according to claim 16 for obtaining an emulsion in which the oily phase comprises a silicone oil, comprising the steps of:
i) on one hand, preparing the silicone phase by means of the mixture of its components,
ii) separately preparing the aqueous phase by means of the mixture of its components, and,
iii)mixing said silicone and aqueous phases and stirring until the silicone emulsion is formed.

19. A cosmetic product comprising a composition according to any of claims 1 to 15.

20. A cosmetic product according to claim 19, furthermore comprising an acceptable cosmetic additive.
